# EUROPEAN PATENT SPECIFICATION

(11) **EP 2 441 450 B1**
(45) Date of publication and mention of the grant of the patent: **14.06.2017**
(21) Application number: 10425334.9
(22) Date of filing: 18.10.2010
(51) Int. Cl.: A61K 31/01, A61K 31/122, A61K 31/353, A61K 31/355, A61K 31/385, A61K 9/48, A61P 5/14

(54) **Antioxidant composition for reducing the oxidative stress and side effects ascribable to treatment with levothyroxine**
Antioxidanszusammensetzung zur Reduzierung von oxidativem Stress und der Nebenwirkungen, die auf die Behandlung mit Levothyroxin zurückzuführen sind
Composition antioxydante pour réduire le stress oxydatif et les effets secondaires attribuables à un traitement avec la lévothyroxine

(43) Date of publication of application: 18.04.2012
(73) Proprietor: BLYMUM S.R.L., 20137 Milano (IT)
(72) Inventor: Cornelli, Umberto, 20129 Milano (IT)
(74) Representative: Cinquantini, Bruno

(56) References cited:
- EP-A1- 0 550 108
- WO-A1-2010/112071
- BIEWENGA GERREKE P ET AL: "The pharmacology of the antioxidant lipoic acid", GENERAL PHARMACOLOGY, PERGAMON PRESS, OXFORD, GB, vol. 29, no. 3, 1 January 1997 (1997-01-01), pages 315-331, XP002596401, ISSN: 0306-3623
- BRUNO A N ET AL: "Activation of adenosine A1 receptors alters behavioral and biochemical parameters in hyperthyroid rats", BEHAVIOURAL BRAIN RESEARCH, ELSEVIER, AMSTERDAM, NL, vol. 167, no. 2, 28 February 2006 (2006-02-28), pages 287-294, XP025031882, ISSN: 0166-4328, DOI: DOI:10.1016/J.BBR.2005.09.017 [retrieved on 2006-02-28]
- SEVEN ARZU ET AL: "Antioxidant status in experimental hyperthyroidism: Effect of vitamin E supplementation", CLINICA CHIMICA ACTA, vol. 256, no. 1, 1996, pages 65-74, XP002623291, ISSN: 0009-8981
- SEVEN A ET AL: "Lipid peroxidation and vitamin E supplementation in experimental hyperthyroidism", CLINICAL CHEMISTRY 1996 US, vol. 42, no. 7, 1996, pages 1118-1119, XP002623292, ISSN: 0009-9147
- SAHOO D K ET AL: "Protective effects of vitamin E and curcumin on l-thyroxine-induced rat testicular oxidative stress", CHEMICO-BIOLOGICAL INTERACTIONS 20081125 IE LNKD- DOI:10.1016/J.CBI.2008.07.009, vol. 176, no. 2-3, 25 November 2008 (2008-11-25), pages 121-128, XP002623293, ISSN: 0009-2797
- CORNELLI ET AL: "Antioxidant use in nutraceuticals", CLINICS IN DERMATOLOGY, J.B. LIPPINCOTT, PHILADELPHIA, PA, US, vol. 27, no. 2, 1 March 2009 (2009-03-01), pages 175-194, XP026000325, ISSN: 0738-081X, DOI: DOI:10.1016/J.CLINDERMATOL.2008.01.010 [retrieved on 2009-01-23]
- WIKTORSKA J A ET AL: "Effects of different antioxidants on lipid peroxidation in brain homogenates, induced by L-thyroxine administration in rats", BIOGENIC AMINES, VNU SCIENCE PRESS, UTRECHT, NL, vol. 21, no. 4, 1 January 2007 (2007-01-01), pages 215-224, XP009144809, ISSN: 0168-8561

## Description

### FIELD OF THE INVENTION

The present invention concerns an antioxidant composition for use in reducing the oxidative stress and side effects in individuals undergoing treatment with levothyroxine according to claim 1. In particular, said composition has proved to be particularly suitable in that, as well as being surprisingly effective, it is extremely well tolerated by the body.

### STATE OF THE ART

The main function of the thyroid gland is to regulate metabolism in the human body through the production of thyroid hormones (Ts), i.e. 3,5,3',5'-tetra-iodo-L-thyronine (or L-thyronine; T₄) and 3,5,3'-tri-iodo-L-thyronine (or iodothyronine; T₃). The production of Ts by the thyroid gland is related to an adequate dietary intake of iodine (I₂).

Iodine (I₂) is an essential element, and in order to be absorbed by the gastrointestinal tract it must be reduced to iodide (I⁻) then actively transported to the thyroid gland where it is re-oxidized (by thyroperodixase or TPO) to form iodo-compounds with amino acids or lipids.

Under normal conditions, I₂ concentration in the blood is very low (0.2 - 0.4 µg/dL, corresponding to about 15-30 nM).

Iodine circulation in plasma is complex since in the blood there circulates iodine derived from absorption, iodine produced by the thyroid (and also by other tissues) as well as iodine from metabolically derived iodide compounds i.e. inactivated or to be eliminated.

As regards transport to the thyroid gland, this aspect is not well known and undoubtedly takes place by various paths, as I₂ is an ultratrace element (found in milligram quantities in the body) with the characteristics of essentiality. It is often lacking in many areas of the earth [1]; as it is a trace element essential to the organism, the human body has developed many different ways for its absorption, distribution and recovery.

In the blood, 95% of I₂ is found in organic form (i.e. bound as half of I₂, hereinafter indicated as I) and about 5% as iodide, i.e. I⁻. The organic form is represented by hormones which are transported by TBG (thyroxine binding globulin) which binds to both T4 and T3, the latter with lower affinity; T4 also binds to transthyretin, the retinol binding protein. If these carriers are saturated, albumin can also bind the Ts, as can HDLs. This ease of transport protects hormones from metabolism and excretion, giving them a very long half-life (6-7 days).

The thyroid very efficiently captures the I with which it comes into contact, by means of a membrane protein, called the sodium-iodide symporter or NIS, to the extent that the ratio between its concentration in the thyroid and in the blood ranges from 20 to 50, even reaching 100 if the gland is activated. Thyroid-stimulating hormone (i.e. thyrotropin or TSH) regulates I capture by the thyroid, acting as a NIS stimulator. A lower concentration of I₂ reserves in the gland actually increases its capture by means of the NIS, whereas an abundance thereof decreases synthesis of NIS.

Once I has reached the thyroid by way of the carriers, it is released, again assuming its I⁻ form, and is oxidized, i.e. returned to the I state. This process is carried out by TPO, which uses, as substrate, H₂O₂ which is actively produced by thyroid cells by the dual oxidases (DUOX1/2) located in the apical pole of the thyrocytes.

The thyrocytes hence also need at their disposal an important antioxidant protection system to limit diffusion of H₂O₂, this being a potent and diffusible oxidant which could generate oxidative stress when produced in excess or unutilized. This protective role against any excess oxidation is carried out by a series of antioxidant enzymes, such as peroxiredoxin (PRDX), catalase and glutathione peroxidase (GPx), but also by a set of supplementary antioxidants (mainly from food).

The I, thus reconstituted, is incorporated into the tyrosyl residues of a glycoprotein, called thyroglobulin, which is the protein constituent of thyroid colloid. This protein is composed of two subunits each of 330 kD; it forms the predominant part of thyroid colloid which is mostly extracellular and makes up a large part of the thyroid mass.

For the halogenation of an aromatic ring, such as that of tyrosine which serves as acceptor of iodine, the latter must be oxidized. This oxidation, which constitutes an activation step, is carried out, as aforementioned, by the heme group-containing enzyme thyroiodoperoxidase (TPO), and uses hydrogen peroxide (H₂O₂) as the oxidant. The enzyme is located on the apical cell membrane and the ensuing reaction is iodination of the thyrosyl group of thyroglobulin to form L-mono-iodotyrosine (MIT) and di-iodotyrosine (DIT), which are inactive compounds from the hormonal viewpoint.

At this stage, H₂O₂ production is essential and is induced by an increase in intracellular Ca²⁺, the latter increasing reactive oxygen species (ROS) concentration due to its activating effect. Since H₂O₂ is more diffusible than the ROSs, its presence on the cell membrane, on which it is captured by TPO, can be assumed.

The increase in cellular Ca²⁺, under physiological conditions of normal homeostasis, is caused by TSH.

In this manner, regulation of I capture and hormone production are subject to a well defined control mechanism.

The subsequent reactions for forming the active hormones, namely T3 and T4, take place by MIT or DIT coupling.

Coupling two DITs forms T4, whereas coupling DIT with MIT forms T3, both of which are bound by peptide linkage to thyroglobulin in the colloid follicles, a hormonal reservoir site.

This occurs by the initial formation of an iodotyrosyl-radical or an ionic radical within the thyroglobulin itself through the action of TPO. In theory, TPO can even form T4 if the substrates for its formation are bound to other proteins, but not with the same efficiency.

Overall, the synthetic capability of these hormones depends on two key factors; the availability of I and that of TSH.

Experimentally, in the case of I deficiency, the T4/T3 ratio ranges from 4/1 to 1/3. This occurs because T3 has a lower content of I, in addition to being the more metabolically active form; the body therefore seeks to maintain the system in balance despite the relative lack of I. Additionally, since T3 is the more metabolically active form, T4 deiodinization also takes place. Therefore, the actual active hormone is T3 which is primarily derived from T4 metabolism. The mechanism by which T3 operates is not fully elucidated. Nevertheless, having reached the intracellular milieu, it binds to a nuclear receptor which in turn binds to specific DNA sequences known as TREs (Thyroid hormone response elements) which trigger gene transcription and then specific protein synthesis. T4 also follows the same sequence of events but with a lower efficiency and incompletely since its ability to modify gene transcription has never been demonstrated. For this reason, T4 is regarded as if it were a pro-hormone.

The inability of the thyroid to produce the necessary amounts of Ts is defined as primary hypothyroidism, this being the most common form. There are also central hypothyroidisms, i.e. due to an inability to produce TSH (secondary hypothyroidism) or due to hypothalamic dysfunction (tertiary hypothyroidism), and finally congenital hypothyroidism, which is the primary cause of cretinism.

There is also sub-clinical hypothyroidism, which is characterised by increased levels of TSH; this is relatively common with a prevalence of between 15% and 25% of the population (the latter in patients > 60 years of age). This form is very subtle and can be masked during pregnancy; it is a prelude to reduced psychomotor development in the newborn, suggesting that during pregnancy all hypothyroidism forms should be treated.

Nodular thyroid disorders are the most common endocrine disease. The prevalence of this disease is between 4 and 7% of the population and increases with age, reaching up to 50% of cases by age 60. In these cases, the onset of thyroid cancer is always feared, occurring in 8-10% of cases and is hence an event of considerable clinical/social importance (in the USA about 22,000 cases are diagnosed every year resulting in a mortality rate of 1000 cases/year).

In patients with thyroid insufficiency, therapy involves administration of L-T4. This is actually a substitution treatment, with the patient consuming that amount of hormone which the body cannot produce itself. The initial dose is small and is then gradually increased until the appropriate dose is reached.

L-T4 is absorbed in a variable and incomplete manner, i.e. from 50% to 80%. Administration of L-T4 produces a biphasic wave characterized by a peak between 2 and 4 hours after administration [2], which then remains relatively stable for up to 12 hours then flexes into a very slow decay phase with a t ½ of approximately 7 days.

A key factor regarding this matter is that the increase in levels until steady state is reached occurs gradually and stabilizes after about one month.

These factors are at the root of the problem of oxidative stress (OS) genesis, produced on administration of L-T4, namely that a wave of possible OS is generated daily at least for the length of time needed to reach a daily peak, i.e. between 2 and 4 hours.

However OS could, at least in theory, also remain constant if the body does not activate the appropriate defences.

The genesis of OS derives from the non-use of H₂O₂ which should be used for the synthesis of Ts.

There is experimental evidence that hyperthyroidism increases OS [3].

Thyroid hormone produces a hypermetabolic state and hence accelerates mitochondrial electron transport resulting in the generation of oxidizing equivalents (also known as reactive species or ROS); these are represented by superoxide (O₂^{•}), H₂O₂ and lastly the hydroxyl radical (OH⁻), they all producing lipid peroxidation and hence OS.

When exogenous L-T4 is consumed e.g. for hypothyroidism treatment, a sudden hypermetabolic state is established, added to which is the non-use of H₂O₂ for Ts synthesis; the ease with which an OS condition is generated can hence be understood.

In hypothyroid individuals, the OS condition appears to be balanced because the hypometabolic state restricts the production of oxidizing equivalents as well as non-use of H₂O₂ available for Ts synthesis. As a result, the OS condition in hypothyroidism can be variable, depending on the prevalence of these two components.

Some authors [4] believe that the presence of goitre, and hence an inflammatory state, increases not only production of reactive species but also the accompanying set of antioxidants, thus allowing the situation to be maintained in balance. In practice, this condition should be assessed on a case by case basis in order to determine it with accuracy.

If the antioxidant capacity under hyper- and hypothyroid conditions is examined experimentally and compared to normal conditions [4], it is found to be reduced in all the tissues. If at the same time the levels of oxidized derivatives (MDA i.e. malonyldialdehyde and hydroperoxide) are analyzed in these same tissues, in the hypothyroid state circulatory levels are found not to differ from normality whereas tissue levels are substantially increased.

To summarize, OS is well defined in hyperthyroidism, whereas in hypothyroidism it depends on the efficiency of the antioxidant network and the inflammatory state, although the antioxidant capacity is significantly lower. Usually, the latter condition is more evidently a cause of OS in men than in women [5].

In any event, the essential point is that OS is generated after exogenous L-T4 administration and can vary in extent and duration depending on the subjective availability of the antioxidant set.

In addition, it has also been found that thyroid hormone administration not only consistently generates OS, but also a number of side effects related to the accelerated metabolism induced by the treatment, such as anxiety/agitation, sweating, palpitations and headaches. Often, these side effects are such as to be disabling to the normal performance of daily activities by the individual, who, in some cases, is obliged to be absent from work.

All these side effects are connected to the sudden increase in hormone levels due to daily L-T4 administration which, having an average t ½ of 7 days as was previously stated, has a tendency to accumulate. This accumulation requires on average 30-35 days of therapy, to reach steady state levels of thyroid hormones (T4 and T3) and to limit secretion of thyroid stimulating hormone (TSH).

There is therefore a strongly felt need to find an effective remedy well tolerated by the body that is able to reduce oxidative stress ascribable to levothyroxine treatment in individuals with hypothyroidism, while simultaneously reducing the side effects generated thereby. The object of the present invention is therefore to provide such a remedy.

### SUMMARY OF THE INVENTION

This object was achieved by the antioxidant composition for use claimed in claim 1, comprising oligomeric proanthocyanidins, lipoic acid, vitamin E, lycopene and astaxanthin.

In fact, as will be evident from the following detailed description and the illustrative and non-limiting working examples, the composition of the invention has surprisingly enabled not only oxidative stress to be reduced but also the side effects experienced in individuals affected by hypothyroidism undergoing treatment with levothyroxine, by means of the appropriate combination of selected components which are highly tolerable to the body.

### DETAILED DESCRIPTION OF THE INVENTION

The present invention therefore relates to an antioxidant composition comprising oligomeric proanthocyanidins, lipoic acid, vitamin E, lycopene and for use according to claim 1. It has actually been found that the synergistic combination of these components enables the oxidative stress associated with levothyroxine consumption in individuals affected by hypothyroidism, to be surprisingly and advantageously reduced, while at the same time reducing the side effects caused by said consumption, as will be seen from the examples that follow.

Preferably, said antioxidant composition comprises 1-15% by weight of oligomeric proanthocyanidins, 5-68% by weight of lipoic acid, 1-15% by weight of vitamin E, 0.05-1% by weight of lycopene and 0.01-0.5% by weight of astaxanthin, on the total weight of the composition.

More preferably, said antioxidant composition comprises 1-5% by weight of oligomeric proanthocyanidins, 5-22% by weight of lipoic acid, 1-5% by weight of vitamin E, 0.05-0.3% by weight of lycopene and 0.01-0.1% by weight of astaxanthin, on the total composition weight.

According to a preferred embodiment, said antioxidant composition comprises 1.34% by weight of oligomeric proanthocyanidins, 6.6% by weight of lipoic acid, 1.34% by weight of vitamin E, 0.08% by weight of lycopene and 0.016% by weight of astaxanthin, on the total composition weight.

According to a particularly preferred embodiment, said antioxidant composition comprises 4% by weight of oligomeric proanthocyanidins, 20% by weight of lipoic acid, 4% by weight of vitamin E, 0.24% by weight of lycopene and 0.05% by weight of astaxanthin, on the total composition weight.

Should it be appropriate, as will be seen from the following examples, to increase the intake of said composition in order to reduce even more markedly the side effects caused by levothyroxine consumption, an antioxidant composition comprising the following is preferred: 4-14% by weight of oligomeric proanthocyanidins, 20-65% by weight of lipoic acid, 4-14% by weight of vitamin E, 0.2-0.9% by weight of lycopene and 0.05-0.3% by weight of astaxanthin, on the total composition weight.

Even more preferably, said antioxidant composition comprises 8-14% by weight of oligomeric proanthocyanidins, 40-65% by weight of lipoic acid, 8-14% by weight of vitamin E, 0.4-0.9% by weight of lycopene and 0.1-0.3% by weight of astaxanthin, on the total composition weight.

According to a preferred embodiment, said antioxidant composition comprises 8% by weight of oligomeric proanthocyanidins, 40% by weight of lipoic acid, 8% by weight of vitamin E, 0.48% by weight of lycopene and 0.1% by weight of astaxanthin, on the total composition weight.

According to a particularly preferred embodiment, said antioxidant composition comprises 12% by weight of oligomeric proanthocyanidins, 60% by weight of lipoic acid, 12% by weight of vitamin E, 0.72% by weight of lycopene and 0.15% by weight of astaxanthin, on the total composition weight.

Preferably, the composition of the invention comprises oligomeric proanthocyanidins in the form of red grape seed extract or maritime pine bark extract.

Optionally, the composition of the invention further comprises pharmaceutically acceptable excipients such as microcrystalline cellulose, lactose, talc, magnesium stearate, calcium stearate, colloidal anhydrous silica, aluminium or magnesium silicate, and dibasic calcium phosphate. The present invention concerns the therapeutic application of the said antioxidant composition.

In particular, the antioxidant composition of the invention is used in a treatment for reducing oxidative stress and simultaneously the side effects ascribable to a treatment with levothyroxine according to claim 1. Indeed, as will be seen from the working examples to follow, the composition of the invention has shown a surprising synergistic effect due to the appropriate combination of various components, which separately did not demonstrate an efficacy suitable for the purposes of the present invention.

Preferably, said antioxidant composition is to be administered orally.

Preferably, the antioxidant composition of the invention is in the form of a single unit dose comprising 5-75 mg of oligomeric proanthocyanidins, 25-340 mg of lipoic acid, 5-75 mg of vitamin E, 0.25-5 mg of lycopene and 0.05-2.5 mg of astaxanthin. Preferably, said unit dose comprises 5-25 mg of oligomeric proanthocyanidins, 25-110 mg of lipoic acid, 5-25 mg of vitamin E, 0.25-1.5 mg of lycopene and 0.05-0.5 mg of astaxanthin.

According to a preferred embodiment, said unit dose comprises 6.7 mg of oligomeric proanthocyanidins, 33 mg of lipoic acid, 6.7 mg of vitamin E, 0.4 mg of lycopene and 0.08 mg of astaxanthin.

According to a particularly preferred embodiment, said unit dose comprises 20 mg of oligomeric proanthocyanidins, 100 mg of lipoic acid, 20 mg of vitamin E, 1.2 mg of lycopene and 0.25 mg of astaxanthin.

As previously mentioned, should it be appropriate to increase the dose in order to reduce even more markedly the side effects caused by levothyroxine consumption, as will be seen from the following examples, a unit dose comprising the following is preferred: 20-55 mg of oligomeric proanthocyanidins, 100-325 mg of lipoic acid, 20-55 mg of vitamin E, 1-4.5 mg of lycopene and 0.25-1.5 mg of astaxanthin.

More preferably, said unit dose comprises 40-55 mg of oligomeric proanthocyanidins, 200-325 mg of lipoic acid, 40-55 mg of vitamin E, 2-4.5 mg of lycopene and 0.5-1.5 mg of astaxanthin.

According to a preferred embodiment, said unit dose comprises 40 mg of oligomeric proanthocyanidins, 200 mg of lipoic acid, 40 mg of vitamin E, 2.4 mg of lycopene and 0.5 mg of astaxanthin.

According to a particularly preferred embodiment, said unit dose comprises 60 mg of oligomeric proanthocyanidins, 300 mg of lipoic acid, 60 mg of vitamin E, 3.6 mg of lycopene and 0.75 mg of astaxanthin.

Working examples of the present invention are hereinafter provided for illustrative and non-limiting purposes.

### EXAMPLES

### Example 1.

### Determination of oxidative stress in relation to hormonal control in hypothyroid subjects

### Admission and exclusion criteria

24 subjects were analyzed (11 M; 13 F) aged between 41 and 61 years, affected by either autoimmune or not autoimmune uni- or plurinodular primary hypothyroidism, treated with L-T4-based hormone replacement therapy and monitored for a period of 30 days.

For female subjects, only those patients who had been in menopause for at least one year who were not on any replacement therapy were admitted. Subjects with a body mass index (BMI) >30 or <20 were not admitted nor those undergoing slimming therapies or weight loss diets. Subjects affected by any type of neoplasma were not admitted, and neither were smokers.

On the other hand, subjects with dislipidemia were admitted, though were limited to those in therapeutic treatment with statins for at least 6 months; the use of other lipid-lowering drugs was considered to be an exclusion criterion. Hypertension was a cause for exclusion even if therapeutically controlled.

In uninodular forms, a biopsy investigation was also undertaken (by needle aspiration) to exclude tumour forms; this investigation was sometimes also required in plurinodular forms if one nodule was much larger in size than the others. For admission, tests for determining autoimmune hypothyroidism were not considered, however subjects with a very pronounced goiter were excluded. Therefore, in general, subjects were affected either by autoimmune or non autoimmune hypothyroidism. All other chronic diseases, as well as the use of any dietary supplement, were criteria for exclusion.

### Treatments

The protocol comprised a treatment with L-T4 (levothyroxine) at a fixed dose of 75 µg/ day, taken in the morning in a fasted state, followed after 30 minutes by breakfast. No other treatments were under way except for statins (simvastatin), which were taken before the main meal.

The treatment with L-T4 was maintained for a period of 30 days.

### Type of study

This was an open monocentric study.

### Variables under examination

Levels of T4, T3 and TSH were analyzed, as well as levels of hydroperoxides (d-ROMs test) as indices of oxidative stress (OS). All the determinations were carried out at three distinct times: baseline, after 15 days of treatment (a delay of no more than 2 days was permitted for the follow-up test, in case the follow-up day fell on a holiday) and after 30 days of treatment, i.e. very close to the steady-state plasma levels of replacement hormones.

Also evaluated were the 4 symptoms considered to be side effects of the hormone replacement therapy, namely: anxious state/agitation, sweating, palpitations and headaches.

These symptoms were recorded daily on the relevant form and were evaluated for their frequency in the observation period. Subjects were given a form on which the presence or absence of the listed symptoms were recorded each day, with a severity index varying from 0 to 4, where 0 represents no symptoms and 4 being the maximum value which implied that the subject should attend the Centre for investigations. Each form covered a period of 15 days, so the value for each symptom could vary from 0 (symptom never present) to 15 (symptom present every day). Also recorded on the same form were the days of discomfort in daily activities, the cause of difficulty in accomplishing daily activities or loss of working days. The measurement at the baseline period, i.e. prior to the treatment, was performed at the start of the diagnostic procedure and for a period of 15 days. In this manner, the magnitude of each of the symptoms possibly present before using the hormone replacement therapy could be noted. Therefore all the tested subjects were subjected to three determinations for all the variables under consideration, the only exception being the days of discomfort. These were recorded for the entire 30 days of treatment.

### Laboratory methods

The levels of T4 were determined on the serum by a radioimmunological method (Diagnostic Products Corporation, Los Angeles); the T3 levels were determined on the serum by a radioimmunological method (Magic T3 Radioimmunoassay, Bayer Corporation Diagnostic Division, Tarrytown NY); the TSH levels were determined immunologically with a TSH Immulite 2000(X) kit; oxidative stress was determined by the d-ROMs test which measured plasma hydroperoxides [10, 11] utilizing the FRAS method (H & D Parma - Italy); the triglyceride and total cholesterol levels were determined by FREE tests (Diacron, Grosseto - Italy) on plasma using dedicated reagents.

All the blood samples were collected between 7.30 and 8.30 am, 4 aliquots of 2 to 5 ml of blood being withdrawn from the brachial vein of the subject who had fasted since the previous evening.

### Statistical analysis

The mean values and standard deviations (SD) were determined on all the data; changes in the variables were analyzed by the Student's t-test for interdependent data. The correlations between d-ROMs values and side effects were also evaluated.

### Compliance

Protocol adherence was determined by counting the remaining tablets of those given to the subjects (blisters with 50 tablets).

### Results

All the subjects completed the study. The general characteristics of the subjects are gathered together in table 1 below.

**Table 1. General baseline characteristics of the 24 subjects prior to treatment with L-T4 (Levothyroxine as Eutirox®)**

| Variable | | Mean values ± SD |
|---|---|---|
| Age | [years] | 52 ± 5.1 |
| Sex | | 11 M;13F |
| Height | [m] | 1.68 ± 0.06 |
| Weight | [kg] | 78 ± 5.7 |
| BMI | [kg /m²] | 27.7 ± 1.90 |
| Total cholesterol | [mg/dL] | 249 ± 25.7 |
| Triglycerides | [mg/dL] | 222 ± 42.4 |
| T4^{a} | [µg/dL] | 2.5 ± 0.71 |
| T3^{a} | [ng/dL] | 58 ± 12.4 |
| TSH^{a} | [µU/dL] | 91 ± 10.6 |
| d-ROMs^{a} | [CARR. U.] | 370 ± 27.7 |

| | | |
|---|---|---|
| ^{a} normal values: T4=5-12 µg/dL; T3=70-185 ng/dL; TSH=0.2-3.5 µU/dL; d-ROMS < 300 CARR.U. | | |

The baseline data were indicative of hypothyroidism, in non-obese overweight subjects with dislipidemia. The latter was characterized by a combination of increased levels of total cholesterol and triglycerides, justifying the use of simvastatin. Over the next 30 days, following treatment with 75 µg/ day of L-T4, levels of Ts, TSH and hydroperoxides as well as the presence of side effects were noted, as shown in Table 2 below, and divided into two periods of 15 days each; the value relating to days of discomfort was for the entire 30 days of observation. It could be seen from examination of the data that treatment with L-T4 compensated for the deficiency of circulating Ts, leading to a reduction in TSH. These factors were indicative of hypothyroidism control. However, also noted was a significant increase in side effects which were present over the whole study period, and caused by the increase in circulatory and tissue hormones following L-T4 administration. These effects ranged from 1 and 3 in magnitude (data not shown). Similarly, a significant increase in OS was noted, which, in the baseline measurement, was already higher than normal values (< 300 CARR. U.). The days of daily discomfort were found to be slightly less than 1/3 of the entire observation period.

**Table 2. Variables of hormonal control and OS in hypothyroid subjects undergoing therapy with L-T4. Mean values ± SD**

| Variable | | Mean values ± SD at the various times | | |
|---|---|---|---|---|
| | | Baseline | 15 days | 30 days |
| T4 | [µg/dL] | 2.5 ± 0.71 | 9.4 ± 1.94 | 14.6 ± 2.13 |
| T3 | [ng/dL] | 58 ± 12.4 | 97 ± 10.7 | 168 ± 17.8 |
| TSH | [µU/dL] | 91.0 ± 10.57 | 28.4 ± 9.74 | 7.9 ± 2.81 |
| d-ROMS test | [CARR. U.] | 370 ± 27.7 | 425 ± 34.6 | 435 ± 39.5 |
| Anxiety/agitation | [I]^{a} | 0.3 ± 0.56 | 13.6 ± 1.25 | 13.6 ± 1.28 |
| Sweating | [I] | 0.3 ± 0.44 | 9.6 ± 1.14 | 10.9 ± 2.12 |
| Palpitations | [I] | 0.2 ± 0.51 | 14.1 ± 0.93 | 14.1 ± 1.15 |
| Headaches | [I] | 0.4 ± 0.50 | 12.2 ± 1.64 | 11.9 ± 1.96 |
| Daily discomfort | [d]^{b} | | | 8.8 ± 0.9 |

| | | | | |
|---|---|---|---|---|
| ^{a} Index giving the number of days (out of 15 days) in which the symptom is present independently of its severity. ^{b} number of days out of 30 days (with limited work activity) | | | | |

### Conclusions

The use of L-T4 at a dose of 75 µg/ day for 30 days enabled a recovery of the hypothyroidism condition, in that the laboratory indices typical of said disease were found to be favourably altered, in most cases up to normal levels. In the majority of the subjects treated, substantial side effects also emerged, such as: anxious state/agitation, sweating, palpitations, headaches, as well as an OS condition, probably all due to an increase in circulating and tissue levels of L-T4. Also noted were a significant number of days characterized by daily discomfort, equal to about 1/3 of the entire observation period.

### Example 2.

### Evaluating the effects of L-T4 in three groups of subjects, each treated with a product of known antioxidant activity or with the composition of the invention

Selection of the comparison product types was based on the need to address the OS in different ways.

OS formation derives from an excessive production of reactive oxidant species (ROS) concomitant with a reduced availability of the antioxidant network.

The latter consists of endogenous antioxidant systems and exogenous antioxidants derived from food or supplements. Also, reactive species production is essentially known to be the result of three biological mechanisms, namely: energy production, responsiveness to stimuli such as inflammation or infection, and lastly, metabolism, e.g. synthesis of biogenic amines or prostaglandins etc. [6].

For these reasons, the selection of comparison products for reducing oxidative stress due to thyroid dysfunction and its treatment, has pointed to different antioxidant types, more specifically known as physiological modulators with antioxidant action.

Based on these considerations, the following known products have been identified: AR Stenovit®, and MF Afragil®, which were compared with the composition of the present invention (briefly referred to hereinafter as "AOX")

### Admission and exclusion criteria

The admission criteria were the same as described in Example 1. Also, the subjects admitted to the study were the same as in Example 1. After 30 days of observation in which they had only taken L-T4, these 24 patients were divided by randomization into three groups, A, B and C, with 8 subjects in each, and subjected to the different treatments.

### Treatments

The subjects in all three groups continued with the L-T4 treatment at the same dose of 75 µg/ day, to be taken in the morning in a fasted state 30 minutes before breakfast.
Group A was treated with AR_{D} Stenovit® administered in single dose vials.
Group B was treated with the composition of the present invention (AOX) administered in capsules.
Group C was treated with MF Afragil® administered as tablets.

All treatments lasted for 30 days at the respective daily doses of 1 vial (Group A), 3 capsules (Group B) and 1 tablet (Group C) to be taken at night before bed. Administration was carried out in the evening so as to avoid the products under study from possibly interfering with the absorption of L-T4, which was taken in the morning. The composition of the three products used is given in Table 3 below.

### Type of study

This was a monocentric randomized open study.

### Variables analyzed

The levels of T4, T3 and TSH were analyzed as well as the levels of hydroperoxides (d-ROMs test) as an index of oxidative stress. The determinations were carried out at three times: baseline i.e. value obtained after 30 days treatment with L-T4 alone, after 45 days and after 60 days of therapy, again using L-T4 associated with one of the aforementioned products for each group.

A delay of no more than 2 days was allowed for the follow-up tests at 15 days and 30 days (in case the follow-up fell on a holiday).

In addition, the presence of the 4 symptoms considered to be side effects of the hormone replacement therapy, as well as the days of daily discomfort were assessed in the same manner as described in Example 1.

### Laboratory methods

The laboratory methods and method of implementation were identical to those described in Example 1.

### Statistical analysis

The mean values and standard deviations (SD) were determined on all the data; in the three groups, the differences between variables were analyzed by analysis of variance for orthogonal contrasts.

### Compliance

Protocol adherence was determined by counting the remaining tablets of those given to the subjects (blisters with 50 tablets of L-T4) or remaining vials (box of 40 vials - Group A) or remaining capsules (10 blisters with 10 capsules - Group B) or remaining tablets (blisters with 40 tablets/ 4-blisters of 10 tablets).

**Table 3. Composition per unit of the products used**

| AR_{D}Stenovit® vial | amount | AOX capsule | amount | MF Afragil® tablet | amount |
|---|---|---|---|---|---|
| Vitamin A | 0.4 mg | Pineol ^{a} | 6.7 mg | Astaxanthin | 0.25 mg |
| Beta-carotene | 0.05 mg | Lipoic acid | 33 mg | Bioflavonoids ^{b} | 33 mg |
| Vitamin B6 | 1 mg | Vitamin E | 6.7 mg | Lycopene | 1 mg |
| Vitamin C | 30 mg | Lycopene | 0.4 mg | Calcium lactate | 358 mg |
| Vitamin E | 15 mg | Astaxanthin | 0.08 mg | Calcium carbonate | 521 mg |
| Coenzyme Q₁₀ | 10 mg | Excipients ^{c} | 453.12 mg | Vitamin D3 | 2.5 mcg |
| Selenium | 48 mcg | | | | |
| Bioflavonoids ^{b} | 30 mg | | | | |
| Zinc | 5 mg | | | | |
| L-cysteine | 10 mg | | | | |

| | | | | | |
|---|---|---|---|---|---|
| ^{a} contains 90 % of oligomeric proanthocyanidins ^{b} Citrus extracts ^{c} magnesium stearate and microcrystalline cellulose | | | | | |

### Results

All the enrolled subjects finished the study and compliance was practically complete.

The general characteristics of the three groups are given in Table 4 below.

As can be seen from Table 4, the subjects undergoing treatment were found to be in a phase of good hormonal control, as is evident from the increase in Ts (T4 and T3) and reduction in TSH levels. At the same time, side effects were also apparent in all the groups, of which anxious state/agitation, headaches and palpitations were well represented. In general, these were felt by the subjects to be decidedly unpleasant and undesirable (up to 3) to the point of often limiting daily function.

In the baseline measurement, the parameters for all the variables of the three groups were not significantly different, with the sole exception of TSH levels, which were slightly higher in Group C than in the other groups.

This isolated difference was however very small and was not considered to be of clinical significance, in that all the other variables relating to hypothyroidism control were virtually comparable.

**Table 4 General baseline characteristics of the three groups of selected subjects. Mean values ± SD.**

| Variables | | Groups | | | Analysis of variance for orthogonal contrasts |
|---|---|---|---|---|---|
| | | A | B | C | |
| Age | [years] | 50 ± 5.5 | 53 ± 4.3 | 54 ± 5.2 | A = B = C |
| Sex | | 3 M; 5F | 4M; 4F | 4M; 4F | |
| Height | [m] | 1.69 ± 0.05 | 1.66 ± 0.07 | 1.69 ± 0.06 | A = B = C |
| Body weight | [Kg] | 80 ± 3.5 | 77 ± 7.5 | 78 ± 5.8 | A = B = C |
| BMI | [Kg/m²] | 27.9 ± 1.207 | 28.8 ± 2.12 | 27.2 ± 2.35 | A = B = C |
| Total cholesterol | [mg/dL] | 246 ± 12.4 | 253 ± 39.7 | 244 ± 19.9 | A = B = C |
| Triglycerides | [mg/dL] | 205 ± 28.9 | 233 ± 56 | 227 ± 37.7 | A = B = C |
| d-ROMs | [CARR. U.] | 451 ± 24.2 | 427 ± 51.6 | 429 ± 38.4 | A = B = C |
| T4 | [µg/dL] | 14.2 ± 2.66 | 15.4 ± 1.09 | 14.2 ± 2.34 | A = B = C |
| T3 | [ng/dL] | 168 ± 26.1 | 171 ± 8.3 | 164 ± 16.1 | A = B = C |
| TSH | [µU/dL] | 7.5 ± 4.09 | 7.9 ± 2.42 | 8.7 ± 1.99 | A = B < C |
| Anxiety/agitation | [I]^{a} | 14.0 ± 1.41 | 13.5 ± 1.41 | 13.4 ± 1.06 | A = B = C |
| Sweating | [I] | 9.4 ± 1.51 | 9.5 ± 1.20 | 9.1 ±1.13 | A = B = C |
| Palpitations | [I] | 14.5 ± 0.76 | 13.6 ± 1.06 | 13.4 ± 1.19 | A = B = C |
| Headaches | [I] | 12.4 ± 1.51 | 11.9 ± 2.42 | 11.4 ± 2.07 | A = B = C |

| | | | | | |
|---|---|---|---|---|---|
| ^{a} Index varies from 0 to 15 | | | | | |

The presence of side effects was found to be very similar in all three groups, also with regard to their severity (data not shown).

The follow-up results compared to the baseline result (for therapy with T-L4 alone) are collected together in Table 5 below.

These data showed that no appreciable differences were found between the groups with regard to hypothyroidism control, measurable with the blood hormone indices (T3, T4, TSH).

OS was significantly reduced in all the groups, though more substantial reductions were advantageously noted with Group B.

However, the strikingly clear finding was the reduction of all side effects in Group B treated with the AOX composition of the invention.

These differences, compared to the other treatments, proved to be highly significant (p<0.001) on analysis of variance for orthogonal contrasts.

The differences in activity were already apparent at the follow-up after 15 days and were also stabilized at the next follow-up at 30 days; they were of similar magnitude for all the side effects under consideration.

### Conclusions

Treatment with L-T4 generates OS and highly undesirable side effects.

While OS could be limited by the daily consumption of known physiological modulators, this was not the case for the side effects which remained disadvantageously unchanged.

With the AOX composition on the other hand, a very satisfactory reduction in OS was surprisingly noted together with an unexpected reduction in the side effects caused by hormone replacement therapy.

**Table 5. Analysis of variables relating to hypothyroid control and the side effects caused by L-T4 administered at the same time as three different antioxidant compositions. Mean values ± SD**

| Variables | | Groups | | | Analysis of variance orthogonal contrasts |
|---|---|---|---|---|---|
| | | A | B | C | |
| | | AR_{D} Stenovit® 1 vial/day | AOX 1 cps/day | MF Afragil® 1 cps/day | |
| Baseline d-ROMs | [CARR. U.] | 451 ± 24.2 | 427 ± 51.6 | 429 ± 38.4 | A = B = C |
| 15 days | | 349 ± 26.3 | 322 ± 29.5 | 350 ± 41.5 | A = B = C |
| 30 days | | 309 ± 20.8 | 295 ± 11.7 | 336 ± 42.8 | A = B = C |
| Baseline T4 | [µg/dL] | 14.2 ± 2.66 | 15.4 ± 1.09 | 14.2 ± 2.34 | A = B = C |
| 15 days | | 14.4 ± 2.88 | 16.0 ± 1.72 | 15.6 ± 1.64 | A = B = C |
| 30 days | | 14.0 ± 2.42 | 15.8 ± 1.08 | 15.7 ± 1.36 | A = B = C |
| Baseline T3 | [ng/dL] | 168 ± 26.1 | 171 ± 8.3 | 164 ± 16.1 | A = B = C |
| 15 days | | 165 ± 29.6 | 177 ± 11.6 | 171 ± 14.0 | A = B = C |
| 30 days | | 164 ± 19.7 | 174 ± 10.2 | 172 ± 12.4 | A = B = C |
| Baseline TSH | [µU/dL] | 7.5 ± 4.09 | 7.9 ± 2.42 | 8.7 ± 1.99 | A = B = C |
| 15 days | | 7.0 ± 4.02 | 7.9 ± 1.87 | 8.1 ± 1.09 | A = B = C |
| 30 days | | 6.9 ± 3.48 | 7.4 ± 2.07 | 7.8 ± 1.62 | A = B = C |
| Baseline Anxiety/agitation | [I]^{a} | 14.0 ± 1.41 | 13.5 ± 1.41 | 13.4 ± 1.06 | A = B = C |
| 15 days | | 13.0 ± 1.31 | 4.0 ± 1.41 | 13.0 ± 1.31 | B <A = C |
| 30 days | | 12.8 ± 0.89 | 2.4 ± 1.69 | 12.8 ± 0.71 | B <A = C |
| Baseline Sweating | [I] | 9.4 ± 1.51 | 9.5 ± 1.20 | 9.1 ± 1.13 | A = B = C |
| 15 days | | 9.0 ± 1.20 | 2.0 ± 0.53 | 9.3 ± 1.04 | B < A = C |
| 30 days | | 10.5 ± 1.51 | 2.0 ± 0.53 | 9.1 ± 0.83 | B < A = C |
| Baseline Palpitations | [I] | 14.5 ± 0.76 | 13.6 ± 1.06 | 13.4 ± 1.19 | A = B = C |
| 15 days | | 12.8 ± 1.98 | 4.4 ± 3.07 | 14.0 ± 0.76 | B < A = C |
| 30 days | | 11.4 ± 1.69 | 3.8 ± 2.49 | 12.5 ± 1.31 | B < A = C |
| Baseline Headaches | [I] | 12.4 ± 1.51 | 11.9 ± 2.42 | 11.4 ± 2.07 | A = B = C |
| 15 days | | 10.9 ± 1.36 | 4.5 ± 1.85 | 11.1 ± 0.99 | B < A = C |
| 30 days | | 10.4 ± 1.51 | 2.1 ± 1.36 | 10.9 ± 1.73 | B < A = C |

| | | | | | |
|---|---|---|---|---|---|
| ^{a} Index varies from 0 to 15 | | | | | |

### Example 3.

### Evaluating the effect of the composition of the present invention (AOX) compared to that of a placebo in a double blind study

Following the results achieved in the previous Example 2, which were obtained in a clinical study undertaken with open methodology, it was considered necessary to confirm the data obtained by performing a double blind study comparing AOX with an identical placebo administered using the identical methodology.

### Admission and exclusion criteria

28 patients of both sexes were analyzed (12 M and 16 F), aged between 39 and 61 years. The selection criteria were identical to those of Example 1, except that patients who were smokers and those with a BMI <25 and TSH levels < 50 µU/dl were excluded. Only concomitant treatments with statins were admitted.

So as to avoid imbalances between the two groups under consideration, particularly for the TSH and BMI variables, a pre-selection of the subjects was carried out.

This pre-selection was conducted on a pool of 45 patients, isolating 28 cases with TSH > 50 µU/dl and BMI >25 and <30. This strategy derived from the previous observations which indicated that subjects outside these parameters demonstrated highly variable hormonal control, and were hence unsuitable.

### Treatments

All the subjects consumed L-T4 at a fixed dose of 75 µg/day administered in the morning in a fasted state 30 minutes before breakfast. The 28 subjects were then divided into two groups: Group A (treatment with placebo) and Group B (treatment with AOX).

Group B was then treated with the AOX composition of the invention, but unlike in Example 2, the daily amount of active principles was inserted into a single capsule instead of being divided into three capsules.

This strategy facilitated treatment compliance.

The content of each capsule is shown in Table 6 below.

**Table 6. Component amounts in one AOX capsule**

| AOX capsule | amount |
|---|---|
| Pineol ^{a} | 20 mg |
| Lipoic acid | 100 mg |
| Vitamin E | 20 mg |
| Lycopene | 1.2 mg |
| Astaxanthin | 0.25 mg |
| Excipients ^{b} | 358.55 mg |

| | |
|---|---|
| ^{a} contains 90% oligomeric proanthocyanidins ^{b} magnesium stearate and microcrystalline cellulose | |

Group A was treated with a placebo, prepared in capsules containing only the excipients used to prepare AOX. Both treatments were administered for a period of 30 days and were taken in the evening to avoid any interference with absorption of L-T4, which was administered in the morning in a fasted state 30 minutes before breakfast. The packaging of the AOX and placebo were indistinguishable.

### Type of study

This was a randomized double blind study; since not all the subjects were being treated with statins, the randomization list was divided such that subjects taking statins were equally represented in both groups.

### Analyzed variables

The levels of T4, T3 and TSH were analyzed as well as the levels of hydroperoxides (d-ROMs test) as an index of oxidative stress. The determinations were carried out at two distinct times: baseline and after 30 days of treatment with L-T4.

For the assessment of side effects i.e. anxiety/agitation, sweating, palpitations, headaches, data were also collected after 15 days of therapy in addition to the data assessed at 30 days.

Also taken into account were the days of "daily discomfort" recorded during the study, i.e. those days when the side effects were the cause of reduced activity and/or absence from work. A delay of no more than 2 days was allowed for the follow-up tests at 15 days and 30 days (in case the follow-up day fell on a holiday). The presence of the 4 side effects of hormonal therapy and the days of daily discomfort were ascertained in the same manner as described in Example 1.

### Laboratory methods

The laboratory methods and methods of implementation were the same as those described in Example 1.

### Statistical analysis

The mean values and standard deviations (SD) were determined on all the data; the differences between the variables in the two groups were analyzed by Student's t-test for independent data.

### Compliance

Protocol adherence was determined by counting the remaining tablets of those given to the patients (blisters with 40 tablets of L-T4) and the remaining capsules (blisters with 40 caps; 4 blisters of 10 caps for both AOX and placebo).

### Results

Four subjects (1 M and 3 F) were excluded from the research because their compliance regarding treatment with L-T4 and the products under consideration was not reliable (large number of tablets and capsules remaining at the follow-up after 30 days).

Therefore, these subjects were excluded from the evaluation, which was hence carried out on 24 cases (11 M and 13 F). These cases were then divided into equal numbers in the two treatment groups (12/ group).

The general characteristics of the two groups at the start of the treatments are given in table 7 below.

**Table 7. General baseline characteristics of the hypothyroid subjects before treatment with L-T4. Mean values ± SD**

| Variables | | Groups | | Student's T-test |
|---|---|---|---|---|
| | | Group A (Placebo) | Group B (AOX) | |
| Age | [years] | 50 ± 4.9 | 52 ± 5.3 | Ns |
| Sex | | 5 M; 7F | 6 M; 6F | |
| Height | [m] | 1.67 ± 0.06 | 1.66 ± 0.073 | Ns |
| Weight | [kg] | 77 ± 5.1 | 75 ± 5.9 | Ns |
| BMI | [kg /m²] | 27.8 ± 1.71 | 27.5 ± 2.99 | Ns |
| Use of statins | | 9/12 | 9/12 | Ns |
| Total cholesterol | [mg/dL] | 266 ± 23.2 | 258 ± 23.1 | Ns |
| Triglycerides | [mg/dL] | 278 ± 37.6 | 285 ± 42.0 | Ns |
| T4 | [µg/dL] | 3.3 ± 0.73 | 2.6 ± 0.70 | Ns |
| T3 | [ng/dL] | 54 ± 13.6 | 62 ± 8.7 | Ns |
| TSH | [µU/dL] | 109.2 ± 35.1 | 96.6 ± 7.23 | Ns |
| d-ROMs | [CARR. U.] | 347 ± 36.2 | 377 ± 23.9 | Ns |

| | | | | |
|---|---|---|---|---|
| Ns = p> 0.05 | | | | |

The essential similarity of the two groups could be noted, also because of the pre-selection, in that no significant differences were found for any of the variables under consideration.

The changes in hormonal status, in OS and the presence of side effects during the 30-day treatment period with L-T4 combined with the products under study are shown in Table 8 below. As can be seen, the two treatments were essentially identical with regard to hormonal control. However, the frequency of side effects and the daily days of discomfort were substantially different, due to the degree of OS.

**Table 8. Changes in hormone and hydroperoxide levels in relation to the appearance of side effects in subjects in therapy with L-T4 in combination with AOX or placebo treatment. Mean values ± SD**

| Variables | | Groups | | Student's t-test |
|---|---|---|---|---|
| | | Group A (AOX 1 cps) | Group B (Placebo 1 cps) | |
| Baseline d-ROMs | [CARR. U.] | 347 ± 36.2 | 377 ± 23.9 | Ns |
| 30 days | | 472 ± 40.9 | 278 ± 11.9 | p < 0.05 |
| Baseline T4 | [µg/dL] | 3.3 ± 0.73 | 2.6 ± 0.70 | Ns |
| 30 days | | 8.9 ± 1.16 | 9.3 ± 1.37 | Ns |
| Baseline T3 | [ng/dL] | 54 ± 13.6 | 62 ± 8.7 | Ns |
| 30 days | | 145 ± 18.7 | 153 ± 15.5 | Ns |
| Baseline TSH | [µU/dL] | 109.2 ± 35.1 | 96.6 ± 7.23 | Ns |
| 30 days | | 2.5 ± 1.59 | 1.9 ± 0.30 | Ns |
| Baseline Anxiety/agitation | [I]^{a} | 0.4 ± 0.67 | 0.1 ± 0.29 | Ns |
| 15 days | | 11.7 ± 2.27 | 3.5 ± 1.24 | p < 0.05 |
| 30 days | | 11.6 ± 2.35 | 2.1 ± 1.24 | p < 0.05 |
| Baseline Sweating | [I] | 0.2 ± 0.39 | 0.4 ± 0.67 | Ns |
| 15 days | | 12.9 ± 1.68 | 3.9 ± 2.54 | p < 0.05 |
| 30 days | | 12.4 ± 1.73 | 3.0 ± 2.45 | p < 0.05 |
| Baseline Palpitations | [I] | 0.5 ± 0.80 | 0.6 ± 0.67 | Ns |
| 15 days | | 14.3 ± 0.78 | 5.1 ± 1.93 | p < 0.05 |
| 30 days | | 14.6 ± 0.79 | 4.1 ± 1.00 | p < 0.05 |
| Baseline Headaches | [I] | 0.2 ± 0.39 | 0.2 ± 0.39 | Ns |
| 15 days | | 11.0 ± 1.76 | 4.2 ± 1.85 | p < 0.05 |
| 30 days | | 12.7 ± 1.83 | 1.8 ± 0.94 | p < 0.05 |
| Daily discomfort | [d]^{b} | 0.9 ± 0.67 | 6.0 ± 1.71 | P < 0.05 |

| | | | | |
|---|---|---|---|---|
| ^{a} Index varies from 0 to 15; ^{b} number of days out of 30 days (with limited work activity) | | | | |

Indeed, the ineffectiveness of the placebo compared to the treatment with the AOX composition was clear for all the assessments made at 15 and 30 days.

Regarding the side effects, the limitation to daily activities was found to be greatly improved by AOX administration, but was found to be markedly and substantially disabling in cases treated with the placebo (an average of 6 days out of the 30 days of monitoring, corresponding to 1/5 of the entire observation period), being also the cause of loss of working days.

### Conclusions

In hypothyroid subjects, the concomitant use of the AOX composition of the invention and L-T4 substantially reduced the level of OS while at the same time highly significantly and advantageously limited the incidence of side effects typical at the start of hormone replacement therapy.

Moreover, therapy with the AOX composition enabled daily activities to be performed more easily and, in particular, more normally.

### Example 4.

### Dose/effect evaluation (dose-finding) of the effect of the composition of the invention on OS and side effects caused by L-T4 therapy

The previous examples demonstrated that, with the AOX composition at the doses used above, it was possible to reduce both the degree of OS and the occurrence of side effects typical at the initial phase of replacement therapy with L-T4.

The aim of this example was to evaluate two different dosages of AOX to determine whether there is a dose/effect relationship and then to determine the best dosage to be used and the most appropriate dose/benefit/cost relationship.

In the previous examples, the amount used of the AOX composition was shown to exhibit a surprising activity. This total dosage was administered either divided into 3 units (3 cps) or as a single unit (1 cps).

### Admission and exclusion criteria

20 hypothyroid subjects were analyzed (8 M and 12 F) for L-T4 therapy, divided into two groups (Group A and Group B respectively) composed of 10 subjects each. The same criteria were used as applied in Example 1.

A pre-selection was also carried out in this example, based on subjects with TSH > 50 µU/dL, and a BMI > 25 but < 30.

### Treatments

For the purpose of conducting this clinical pharmacology study of dose/effect analysis, capsules were used in this example with contents as reported in Example 1, with a different dosage being administered to each respective group. All participants in the study were treated with 75 µg/day of L-T4 for a 30 day period. The patients were allocated to one of the two groups according to a randomization scheme.

Each group was treated with the following AOX dosages respectively, to be taken at night before going to bed:
- Group A: 3 capsules;
- Group B: 2 capsules;
with each of said capsules having the composition given in Table 3.

The treatment with L-T4 instead took place in the morning 30 minutes before breakfast.

### Type of study

This was a monocentric randomized double blind experimental protocol.

### Analyzed variables

The levels of T4, T3 and TSH were analyzed as well as the levels of hydroperoxides (d-ROMs test) as an index of oxidative stress. The determinations were carried out at three distinct times: baseline, after 15 days and after 30 days of treatment with L-T4.

The side effects, such as anxiety/agitation, sweating, palpitations, headaches, were also assessed at the same times, and their severity was also recorded. The days of "daily discomfort" were also assessed.

A delay of no more than 2 days was allowed for the follow-up tests at 15 days and 30 days (in case the follow-up day fell on a holiday).

The presence of the 4 symptoms considered to be side effects of the hormone replacement therapy was ascertained in the same manner as described in Example 1.

### Laboratory methods

The laboratory methods and methods of implementation were the same as those described in Example 1.

### Statistical analysis

The mean values and standard deviations (SD) were determined on all the data; the differences between the variables in the two groups were analyzed by analysis of variance.

### Compliance

Protocol adherence was determined by counting the remaining tablets of those given to the subjects (blisters with 40 tablets of L-T4) and the remaining capsules (blisters of 10 caps).

### Results

All the subjects completed the study. The general characteristics of the 20 subjects analyzed are given in the following Table 9.

**Table 9. General baseline characteristics of the two groups of subjects undergoing treatment with L-T4 in combination with different AOX dosages. Mean values ± SD.**

| Variables | | Groups | | Analysis of variance |
|---|---|---|---|---|
| | | Group A 3 cps AOX | Group B 2 cps AOX | |
| Age | [years] | 51 ± 3.2 | 52 ± 3.9 | A = B |
| Sex | | 4 M; 6F | 4 M; 6F | |
| Height | [m] | 1.68 ± 0.06 | 1.69 ± 0.05 | A = B |
| Body weight | [Kg] | 77 ± 2.9 | 78 ± 4.5 | A = B |
| BMI | [Kg/m²] | 27.6 ± 1.65 | 27.2 ± 1.52 | A = B |
| Total cholesterol | [mg/dL] | 242 ± 30.9 | 244 ± 20.0 | A = B |
| Triglycerides | [mg/dL] | 222 ± 33.3 | 229 ± 34.4 | A = B |
| d-ROMs | [CARR. U.] | 391 ± 22.7 | 359 ± 31.2 | A = B |
| T4 | [µg/dL] | 3.2 ± 0.57 | 2.6 ± 0.52 | A = B |
| T3 | [ng/dL] | 50 ± 8.4 | 56 ± 10.3 | A = B |
| TSH | [µU/dL] | 101.0 ± 28.60 | 113.8 ± 25.19 | A = B |
| Anxiety/agitation | [I]^{a} | 0.5 ± 0.53 | 0.1 ± 0.32 | A = B |
| Sweating | [I] | 0.3 ± 0.48 | 0.5 ± 0.53 | A = B |
| Palpitations | [I] | 0.0 ± 0.00 | 0.0 ± 0.00 | A = B |
| Headaches | [I] | 0.1 ± 0.32 | 0.0 ± 0.00 | A = B |

| | | | | |
|---|---|---|---|---|
| ^{a} Index varies from 0 to 15 | | | | |

From analysis of the data, it can be concluded that the two groups were comparable for all the variables under consideration, as the analysis of variance showed there was no significant mean differences. The follow-up tests carried out in the two periods separated by 15 days showed some significant differences, both for the OS test and the frequency of side effects.

The results are gathered together in Table 10 below.

From examination of table 10, it was noted that the parameters for hormonal control were indistinguishable between the two groups (analysis of variance with p > 0.05). Also, OS was found to be reduced in both the groups.

Also with regard to the side effects (scored between 2 and 3 in magnitude), an improvement was noted for both Group A and Group B.

However, for both headaches and work activities the most significant improvement was found in Group A.

Therefore, on the whole, a dose dependency was noted, for which the best results were obtained by increasing the dosages.

**Table 10. Analysis of the variables relating to hypothyroidism control and side effects caused by L-T4 administered at the same time as two different dosages (2 or 3 cps/day) of AOX. Mean values ± SD.**

| Variables | | Groups | | Analysis of variance. Orthogonal contrasts |
|---|---|---|---|---|
| | | Group A 3 cps AOX | Group B 2 cps AOX | |
| Baseline d-ROMs | [CARR. U.] | 391 ± 22.7 | 359 ± 31.2 | A = B |
| 15 days | | 288 ± 38.4 | 386 ± 24.5 | A < B |
| 30 days | | 268 ± 20.9 | 365 ± 39.1 | A < B |
| Baseline T4 | [µg/dL] | 3.2 ± 0.57 | 2.6 ± 0.52 | A = B |
| 15 days | | 8.4 ± 0.99 | 7.3 ± 0.80 | A = B |
| 30 days | | 10.2 ± 1.43 | 10.6 ± 1.65 | A = B |
| Baseline T3 | [ng/dL] | 50 ± 8.4 | 56 ± 10.3 | A = B |
| 15 days | | 98 ± 14.6 | 107 ± 23.1 | A = B |
| 30 days | | 160 ± 20.2 | 169 ± 10.5 | A = B |
| Baseline TSH | [µtU/dL] | 101.0 ± 28.60 | 113.8 ± 25.19 | A = B |
| 15 days | | 34.2 ± 10.49 | 48.3 ± 13.73 | A = B |
| 30 days | | 2.5 ± 0.51 | 2.4 ± 0.57 | A = B |
| Baseline Anxiety/agitation | [I]^{a} | 0.5 ± 0.53 | 8.1 ± 0.74 | A = B |
| 15 days | | 0.4 ± 0.51 | 7.8 ± 1.14 | A< B |
| 30 days | | 0.5 ± 0.53 | 2.4 ± 1.69 | A< B |
| Baseline Sweating | [I] | 0.3 ± 0.48 | 0.5 ± 0.53 | A = B |
| 15 days | | 0.3 ± 0.48 | 8.5 ± 2.27 | A < B |
| 30 days | | 0.5 ± 0.71 | 8.3 ± 1.16 | A < B |
| Baseline Palpitations | [I] | 0.0 ± 0.00 | 0.0 ± 0.00 | A = B |
| 15 days | | 0.3 ± 0.48 | 9.1 ± 1.97 | A < B |
| 30 days | | 0.4 ± 0.52 | 9.0 ± 1.33 | A < B |
| Baseline Headaches | [I] | 0.1 ± 0.32 | 0.0 ± 0.00 | A = B |
| 15 days | | 0.6 ± 0.52 | 11.9 ± 2.18 | A < B |
| 30 days | | 0.6 ± 0.52 | 12.5 ± 1.43 | A < B |
| Daily discomfort ^{b} | | 0.6 ± 0.70 | 3.3 ± 1.16 | A < B |

| | | | | |
|---|---|---|---|---|
| ^{a} Index varies from 0 to 15; ^{b} number of days out of 30 days (with limited work activity) | | | | |

### Conclusions

A dose dependency was noted in this study, in that an AOX dosage of 3 cps showed improved activity compared to that found with 2 cps, hence the higher dose was preferable.

### Example 5.

### Evaluating the action of certain components of the composition of the invention on OS and the side effects of L-T4, compared to the synergy of the complete composition

In the previous Examples, the composition of the present invention (AOX) was shown to be active in reducing OS induced by treatment with L-T4 as well as the side effects ascribable thereto.

With the aim of testing the synergy between the compounds in the composition of the invention, the effects of only some of said compounds were analyzed. In particular, the oligomeric proanthocyanidins were tested alone so as to evaluate their effectiveness as such. In addition, the other components of AOX were also analyzed, i.e. astaxanthin, lipoic acid, vitamin E and lycopene.

The results of these tests were compared with the results obtained with the complete AOX composition.

### Admission and exclusion criteria

30 hypothyroid subjects were analyzed (15 M and 15 F) for L-T4 therapy, divided into three groups (Group A, Group B and Group C respectively) each composed of 10 subjects. The same criteria applied in Example 1 were used.

A pre-selection was also carried out in this example based on subjects with TSH > 50 µU/dl and BMI >25 but <30.

### Treatments

All the research participants were treated with 75 µg/day of L-T4 for a 30 day period. The patients were allocated to one of the Groups A, B or C according to a randomization scheme.

Group A was treated with the complete AOX composition at a dose of 1 cps/day, Group B was treated with oligomeric proanthocyanidins alone, while Group C was treated with astaxanthin, vitamin E, lycopene and lipoic acid. The three compositions are given in Table 11 below, they being indistinguishable from each other. The treatment was continued for a 30 day period with evening consumption before bedtime. Treatment with the L-T4 was instead carried out in the morning 30 minutes before breakfast.

### Type of study

This was a monocentric randomized double blind experimental protocol.

### Analyzed variables

The levels of T4, T3 and TSH were analyzed as well as the levels of hydroperoxides (d-ROMs test) as an index of oxidative stress. The determinations were carried out at three distinct times: baseline, after 15 days and after 30 days of treatment with L-T4.

The side effects, such as anxiety/agitation, sweating, palpitations, headaches, were also assessed at the same times. The days of discomfort leading to limited daily or work activities over the 30 day period were also assessed.

A delay of no more than 2 days was allowed for the follow-up tests at 15 days and 30 days (in case the follow-up day fell on a holiday).

The presence of the 4 symptoms and daily discomfort were recorded with the same methods as described in Example 1.

### Laboratory methods

The laboratory methods and methods of implementation were identical to those described in Example 1.

### Statistical analysis

The mean values and standard deviations (SD) were determined on all the data; the differences between the variables in the two groups were analyzed by analysis of variance for orthogonal contrasts.

### Compliance

Protocol adherence was determined by counting the remaining tablets of those given to the subjects (blisters with 40 tablets of L-T4) and the remaining capsules (4 blisters of 10 caps for each type of treatment).

**Table 11. Partial compositions and AOX composition used in hypothyroid subjects treated with 75 µg/day of L-T4**

| Group A | | Group B | | Group C | |
|---|---|---|---|---|---|
| AOX [1 cps] | | Partial 1 [1 cps] | | Partial 2 [1 cps] | |
| | amount | | amount | | amount |
| Pineol^{a} | 20 mg | Pineol^{a} | 20 mg | Lipoic acid | 100 mg |
| Lipoic acid | 100 mg | Excipients ^{b} | 480 mg | Vitamin E | 20 mg |
| Vitamin E | 20 mg | | | Lycopene | 1.2 mg |
| Lycopene | 1.2 mg | | | Astaxanthin | 0.25 mg |
| Astaxanthin | 0.25 mg | | | Excipients ^{b} | 378.55 mg |
| Excipients ^{b} | 358.55 mg | | | | |
| TOTAL | 500 mg | | 500 mg | | 500 mg |

| | | | | | |
|---|---|---|---|---|---|
| ^{a} contains 90% oligomeric proanthocyanidins ^{b} magnesium stearate, silica, dibasic calcium phosphate, talc | | | | | |

### Results

All subjects finished the experimental period. The variables relating to the general characteristics of the three groups are given in Table 12 below.

**Table 12. General baseline characteristics of the three groups of subjects under treatment with L-T4 in combination with a partial composition or with the AOX composition. Mean values ± SD**

| Variables | | Groups | | | Analysis of variance. Orthogonal contrasts |
|---|---|---|---|---|---|
| | | Group A [AOX] | Group B [Partial 1] | Group C [Partial 2] | |
| Age | [years] | 52 ± 3.6 | 51 ± 6.63 | 53 ± 6.20 | A = B = C |
| Sex | | 5 M; 5 F | 4 M; 6F | 4 M; 6F | |
| Height | [m] | 1.70 ± 0.05 | 1.69 ± 0.07 | 1.69 ± 0.05 | A = B = C |
| Body weight | [Kg] | 78 ± 3.7 | 76 ± 5.9 | 79 ± 4.0 | A = B = C |
| BMI | [Kg/m²] | 27.1 ± 1.99 | 26.6 ± 1.31 | 27.6 ± 1.67 | A = B = C |
| Total cholesterol | [mg/dL] | 269 ± 21.2 | 268 ± 21.9 | 258 ± 23.4 | A = B = C |
| Triglycerides | [mg/dL] | 264 ± 25.4 | 255 ± 15.4 | 288 ± 44.6 | A = B = C |
| d-ROMs | [CARR. U.] | 387 ± 43.9 | 363 ± 29.1 | 364 ± 12.7 | A = B = C |
| T4 | [µg/dL] | 3.2 ± 0.61 | 2.6 ± 0.66 | 2.6 ± 0.86 | A = B = C |
| T3 | [ng/dL] | 50 ± 8.5 | 46 ± 5.8 | 47 ± 4.9 | A = B = C |
| TSH | [µU/dL] | 101.4 ± 28.59 | 115.0 ± 24.46 | 116.6 ± 14.09 | A = B = C |
| Anxiety/agitation | [I]^{a} | 0.3 ± 0.48 | 0.1 ± 0.32 | 0.4 ± 0.52 | A = B = C |
| Sweating | [I] | 0.1 ± 0.32 | 0.3 ± 0.48 | 0.5 ± 0.53 | A = B = C |
| Palpitations | [I] | 0.0 ± 0.00 | 0.0 ± 0.00 | 0.0 ± 0.00 | A = B = C |
| Headaches | [I] | 0.2 ± 0.42 | 0.0 ± 0.00 | 0.0 ± 0.00 | A = B = C |

| | | | | | |
|---|---|---|---|---|---|
| ^{a} Index varies from 0 to 15 | | | | | |

From Table 12 it could be seen that the groups subjected to the study were essentially comparable as the analysis of variance had in no case revealed differences worthy of note. The data were consistent with those of subjects affected by primary hypothyroidism, overweight and mild dislipidemia (in any event treated with statins).

Treatment with the three analyzed compositions produced the side effects as shown in the following table 13.

**Table 13. Analysis of the variables relating to hypothyroidism control and the side effects caused by L-T4 administered at the same time as one of the three compositions**

| Variables | | Groups | | | Analysis of variance. Orthogonal contrasts |
|---|---|---|---|---|---|
| | | A | B | C | |
| | | AOX | Partial 1 | Partial 2 | |
| Baseline d-ROMs | [CARR. U.] | 387 ± 43.9 | 363 ± 29.1 | 374 ± 24.6 | A = B = C |
| 30 days | | 279 ± 27.9 | 440 ± 46.0 | 421 ± 26.2 | A < B = C |
| Baseline T4 | [µg/dL] | 3.2 ± 0.61 | 2.6 ± 0.66 | 2.6 ± 0.86 | A = B = C |
| 30 days | | 10.2 ± 1.33 | 10.0 ± 1.29 | 11.0 ± 2.23 | A = B = C |
| Baseline T3 | [ng/dL] | 50 ± 8.5 | 46 ± 5.8 | 47 ± 4.9 | A = B = C |
| 30 days | | 159 ± 20.2 | 176 ± 12.8 | 175 ± 13.0 | A = B = C |
| Baseline TSH | [µU/dL] | 101.4 ± 28.59 | 115.0 ± 24.46 | 116.6 ± 14.09 | A = B = C |
| 30 days | | 2.4 ± 0.54 | 2.5 ± 0.77 | 1.9 ± 0.38 | A = B = C |
| Baseline Anxiety/agitation | [I]^{a} | 0.3 ± 0.48 | 0.1 ± 0.31 | 0.4 ± 0.52 | A = B = C |
| 15 days | | 0.3 ± 0.48 | 10.1 ± 1.66 | 10.4 ± 1.35 | A< B = C |
| 30 days | | 0.4 ± 0.52 | 9.8 ± 1.54 | 10.3 ± 1.57 | A< B < C |
| Baseline Sweating | [I] | 0.1 ± 0.32 | 0.3 ± 0.48 | 0.5 ± 0.53 | A = B = C |
| 15 days | | 0.2 ± 0.42 | 9.8 ± 1.87 | 10.4 ± 2.01 | A <B = C |
| 30 days | | 0.4 ± 0.70 | 10.2 ± 1.87 | 10.2 ± 1.81 | A <B < C |
| Baseline Palpitations | [I] | 0.0 ± 0.00 | 0.0 ± 0.00 | 0.0 ± 0.00 | A = B = C |
| 15 days | | 0.0 ± 0.00 | 13.4 ± 1.26 | 13.0 ± 1.70 | A <B = C |
| 30 days | | 0.3 ± 0.48 | 11.7 ± 2.36 | 10.8 ± 4.31 | A <B = C |
| Baseline Headaches | [I] | 0.2 ± 0.42 | 0.0 ± 0.00 | 0.0 ± 0.00 | A = B = C |
| 15 days | | 0.4 ± 0.52 | 12.6 ± 1.71 | 13.1 ± 1.05 | A <B = C |
| 30 days | | 0.0 ± 0.00 | 12.5 ± 1.90 | 10.8 ± 4.31 | A <B = C |
| Daily discomfort | [d]^{b} | 1.1 ± 0.57 | 5.9 ± 1.29 | 5.7 ± 0.82 | A <B = C |

| | | | | | |
|---|---|---|---|---|---|
| ^{a} Index varies from 0 to 15; ^{b} number of days out of 30 days (with limited work activity) | | | | | |

From examination of Table 13 no distinguishable changes were noted in the three Groups for any of the variables relating to hypothyroidism control (T3, T4, TSH); in all cases in the three Groups a highly demonstrable control could be noted from the mean values given.

Regarding the side effects, the partial formulations did not alter the extent of the various parameters which were instead surprisingly and advantageously highly significantly decreased by the complete AOX composition.

In particular, there was a significant difference regarding the days of discomfort, with only the AOX composition being found able to substantially and rapidly improve the situation.

### Conclusions

In subjects with hypothyroidism undergoing initial treatment with L-T4, the use of oligomeric proanthocyanidins alone (partial 1) or the other 4 compounds alone (partial 2) or the complete composition (AOX) did not interfere with hormonal control.

With regard to the presence of side effects and OS, only the complete AOX composition was able to reduce them significantly. These positive effects of AOX, which were not encountered with its separate components, were also noted for the daily activities.

Therefore, the synergistic effect between the components of the composition of the invention has been demonstrated, enabling not only OS ascribable to levothyroxine treatment to effectively reduced, but also the side effects that manifest in the same situation.

### Bibliography

[1] Dunn JT. Iodine. In: Modern nutrition in health and diseases. Tenth edition-Lippincott Williams & Wilkins 2006:300.
[2] Blakesley VA. Current methodology to assess bioequivalence of levothyroxine sodium products is inadequate. AAPS J (American Association Pharmaceutical Scientists) 2005;7:E42-E46.
[3] Venditti P, Balestrieri M, Di Meo S, De leo T. Effect of thyroid state on lipid peroxidation, antioxidant defences, and susceptibility to oxidative stress in rat tissue. J Endocrinol 1997;155:151-157.
[4] Poncin S, Gérard AC, Boucquey M et al. Oxidative stress in the thyroid gland: from harmlessness to hazard depending on iodine content. Endocrinology 2008;149.424-433.
[5] Nabda N, Bobby Z, Hamide A. Oxidative stress and protein glycation in primary hypothyroidism. Male/female difference. Clin Exp Med 2008;8:101-108. Cornelli U. Antioxidant use in nutraceuticals. Clin Dermat 2009;27:175-194.
[6] Cesarone MR, Belcaro G, Carratelli M et al. A simple test to monitor oxidative stress. Intern Angiol 1999;18:127-130.

## Claims

1. An antioxidant composition comprising oligomeric proanthocyanidins, lipoic acid, vitamin E, lycopene and astaxanthin for use in the treatment of oxidative stress and the side affects ascribable to treatment with levothyroxine in individuals affected by hypothyroidism.

2. The antioxidant composition for use of claim 1, comprising 1-15% by weight of oligomeric proanthocyanidins, 5-68% by weight of lipoic acid, 1-15% by weight of vitamin E, 0.05-1% by weight of lycopene and 0.01-0.5% by weight of astaxanthin, on the total composition weight.

3. The antioxidant composition for use of claim 2, comprising 1-5% by weight of oligomeric proanthocyanidins, 5-22% by weight of lipoic acid, 1-5% by weight of vitamin E, 0.05-0.3% by weight of lycopene and 0.01-0.1% by weight of astaxanthin, on the total composition weight.

4. The antioxidant composition for use of claim 3, comprising 1.34% by weight of oligomeric proanthocyanidins, 6.6% by weight of lipoic acid, 1.34% by weight of vitamin E, 0.08% by weight of lycopene and 0.016% by weight of astaxanthin, on the total composition weight.

5. The antioxidant composition for use of claim 3, comprising 4% by weight of oligomeric proanthocyanidins, 20% by weight of lipoic acid, 4% by weight of vitamin E, 0.24% by weight of lycopene and 0.05% by weight of astaxanthin, on the total composition weight.

6. The antioxidant composition for use of claim 2, comprising 4-14% by weight of oligomeric proanthocyanidins, 20-65% by weight of lipoic acid, 4-14% by weight of vitamin E, 0.2-0.9% by weight of lycopene and 0.05-0.3% by weight of astaxanthin, on the total composition weight.

7. The antioxidant composition for use of claim 6, comprising 8-14% by weight of oligomeric proanthocyanidins, 40-65% by weight of lipoic acid, 8-14% by weight of vitamin E, 0.4-0.9% by weight of lycopene and 0.1-0.3% by weight of astaxanthin, on the total composition weight.

8. The antioxidant composition for use of any one of claims 1-7, wherein said oligomeric proanthocyanidins are in the form of an extract of red grape seeds or an extract of maritime pine bark.

9. The antioxidant composition for use of claim 1 in the form of a unit dose comprising 5-75 mg of oligomeric proanthocyanidins, 25-340 mg of lipoic acid, 5-75 mg of vitamin E, 0.25-5 mg of lycopene and 0.05-2.5 mg of astaxanthin.

10. The antioxidant composition for use of claim 9 in the form of a unit dose comprising 5-25 mg of oligomeric proanthocyanidins, 25-110 mg of lipoic acid, 5-25 mg of vitamin E, 0.25-1.5 mg of lycopene and 0.05-0.5 mg of astaxanthin.

11. The antioxidant composition for use of claim 10 in the form of a unit dose comprising 6.7 mg of oligomeric proanthocyanidins, 33 mg of lipoic acid, 6.7 mg of vitamin E, 0.4 mg of lycopene and 0.08 mg of astaxanthin.

12. The antioxidant composition for use of claim 10 in the form of a unit dose comprising 20 mg of oligomeric proanthocyanidins, 100 mg of lipoic acid, 20 mg of vitamin E, 1.2 mg of lycopene and 0.25 mg of astaxanthin.

13. The antioxidant composition for use of claim 9 in the form of a unit dose comprising 20-55 mg of oligomeric proanthocyanidins, 100-325 mg of lipoic acid, 20-55 mg of vitamin E, 1-4.5 mg of lycopene and 0.25-1.5 mg of astaxanthin.

## Patentansprüche

1. Antioxidanszusammensetzung aufweisend oligomere Proanthocyanidine, Liponsäure, Vitamin E, Lycopen und Astaxanthin zur Verwendung bei der Behandlung von oxidativem Stress und den Nebenwirkungen, die bei Personen die von Hypothyreose betroffen sind, auf die Behandlung mit Levothyroxin zurückzuführen sind.

2. Antioxidanszusammensetzung zur Verwendung gemäß Anspruch 1, aufweisend 1 - 15 Gew.-% oligomeren Proanthocyanidinen, 5 - 68 Gew.-% Liponsäure, 1 - 15 Gew.-% Vitamin E, 0,05 - 1 Gew.-% Lycopen und 0,01 - 0,5 Gew.-% Astaxanthin am Gesamtgewicht der Zusammensetzung.

3. Antioxidanszusammensetzung zur Verwendung gemäß Anspruch 2, aufweisend 1 - 5 Gew.-% oligomeren Proanthocyanidinen, 5 - 22 Gew.-% Liponsäure, 1 - 5 Gew.-% Vitamin E, 0,05 - 0,3 Gew.-% Lycopen und 0,01 - 0,1 Gew.-% Astaxanthin am Gesamtgewicht der Zusammensetzung.

4. Antioxidanszusammensetzung zur Verwendung gemäß Anspruch 3, aufweisend 1,34 Gew.-% oligomeren Proanthocyanidinen, 6,6 Gew.-% Liponsäure, 1,34 Gew.-% Vitamin E, 0,08 Gew.-% Lycopen und 0,016 Gew.-% Astaxanthin am Gesamtgewicht der Zusammensetzung.

5. Antioxidanszusammensetzung zur Verwendung gemäß Anspruch 3, aufweisend 4 Gew.-% oligomeren Proanthocyanidinen, 20 Gew.-% Liponsäure, 4 Gew.-% Vitamin E, 0,24 Gew.-% Lycopen und 0,05 Gew.-% Astaxanthin am Gesamtgewicht der Zusammensetzung.

6. Antioxidanszusammensetzung zur Verwendung gemäß Anspruch 2, aufweisend 4 - 14 Gew.-% oligomeren Proanthocyanidinen, 20 - 65 Gew.-% Liponsäure, 4 - 14 Gew.-% Vitamin E, 0,2 - 0,9 Gew.-% Lycopen und 0,05 - 0,3 Gew.-% Astaxanthin am Gesamtgewicht der Zusammensetzung.

7. Antioxidanszusammensetzung zur Verwendung gemäß Anspruch 6, aufweisend 8 - 14 Gew.-% oligomeren Proanthocyanidinen, 40 - 65 Gew.-% Liponsäure, 8 - 14 Gew.-% Vitamin E, 0,4 - 0,9 Gew.-% Lycopen und 0,1 - 0,3 Gew.-% Astaxanthin am Gesamtgewicht der Zusammensetzung.

8. Antioxidanszusammensetzung zur Verwendung gemäß einem der Ansprüche 1 bis 7, wobei die oligomeren Proanthocyanidine in Form eines Extrakts aus Kernen von roten Trauben oder eines Extrakts aus der Rinde der Seestrandkiefer vorliegt.

9. Antioxidanszusammensetzung zur Verwendung gemäß Anspruch 1, in Form einer Einzeldosis aufweisend 5 - 75 mg oligomeren Proanthocyanidinen, 25 - 340 mg Liponsäure, 5 - 75 mg Vitamin E, 0,25 - 5 mg Lycopen und 0,05 - 2,5 mg Astaxanthin.

10. Antioxidanszusammensetzung zur Verwendung gemäß Anspruch 9, in Form einer Einzeldosis aufweisend 5 - 25 mg oligomeren Proanthocyanidinen, 25 - 110 mg Liponsäure, 5 - 25 mg Vitamin E, 0,25 - 1,5 mg Lycopen und 0,05 - 0,5 mg Astaxanthin.

11. Antioxidanszusammensetzung zur Verwendung gemäß Anspruch 10, in Form einer Einzeldosis aufweisend 6,7 mg oligomeren Proanthocyanidinen, 33 mg Liponsäure, 6,7 mg Vitamin E, 0,4 mg Lycopen und 0,08 mg Astaxanthin.

12. Antioxidanszusammensetzung zur Verwendung gemäß Anspruch 10, in Form einer Einzeldosis aufweisend 20 mg oligomeren Proanthocyanidinen, 100 mg Liponsäure, 20 mg Vitamin E, 1,2 mg Lycopen und 0,25 mg Astaxanthin.

13. Antioxidanszusammensetzung zur Verwendung gemäß Anspruch 9, in Form einer Einzeldosis aufweisend 20 - 55 mg oligomeren Proanthocyanidinen, 100 - 325 mg Liponsäure, 20 - 55 mg Vitamin E, 1 - 4,5 mg Lycopen und 0,25 - 1,5 mg Astaxanthin.

## Revendications

1. Composition antioxydante comprenant des proanthocyanidines oligomères, de l'acide lipoïque, de la vitamine E, du lycopène et de l'astaxanthine pour son utilisation dans le traitement d'un stress oxydatif et des effets secondaires imputables à un traitement avec la lévothyroxine chez des individus souffrant d'hypothyroïdisme.

2. Composition antioxydante pour son utilisation selon la revendication 1, comprenant 1 à 15 % en poids de proanthocyanidines oligomères, 5 à 68 % en poids d'acide lipoïque, 1 à 15 % en poids de vitamine E, 0,05 à 1 % en poids de lycopène et 0,01 à 0,5 % en poids d'astaxanthine, sur la base du poids total de la composition.

3. Composition antioxydante pour son utilisation selon la revendication 2, comprenant 1 à 5 % en poids de proanthocyanidines oligomères, 5 à 22 % en poids d'acide lipoïque, 1 à 5 % en poids de vitamine E, 0,05 à 0,3 % en poids de lycopène et 0,01 à 0,1 % en poids d'astaxanthine, sur la base du poids total de la composition.

4. Composition antioxydante pour son utilisation selon la revendication 3, comprenant 1,34 % en poids de proanthocyanidines oligomères, 6,6 % en poids d'acide lipoïque, 1,34 % en poids de vitamine E, 0,08 % en poids de lycopène et 0,016 % en poids d'astaxanthine, sur la base du poids total de la composition.

5. Composition antioxydante pour son utilisation selon la revendication 3, comprenant 4 % en poids de proanthocyanidines oligomères, 20 % en poids d'acide lipoïque, 4 % en poids de vitamine E, 0,24 % en poids de lycopène et 0,05 % en poids d'astaxanthine, sur la base du poids total de la composition.

6. Composition antioxydante pour son utilisation selon la revendication 2, comprenant 4 à 14 % en poids de proanthocyanidines oligomères, 20 à 65 % en poids d'acide lipoïque, 4 à 14 % en poids de vitamine E, 0,2 à 0,9 % en poids de lycopène et 0,05 à 0,3 % en poids d'astaxanthine, sur la base du poids total de la composition.

7. Composition antioxydante pour son utilisation selon la revendication 6, comprenant 8 à 14 % en poids de proanthocyanidines oligomères, 40 à 65 % en poids d'acide lipoïque, 8 à 14 % en poids de vitamine E, 0,4 à 0,9 % en poids de lycopène et 0,1 à 0,3 % en poids d'astaxanthine, sur la base du poids total de la composition.

8. Composition antioxydante pour son utilisation selon l'une quelconque des revendications 1 à 7, dans laquelle lesdites proanthocyanidines oligomères sont sous la forme d'un extrait de pépins de raisin rouge ou d'un extrait d'écorce de pin maritime.

9. Composition antioxydante pour son utilisation selon la revendication 1, sous la forme d'une dose unitaire comprenant 5 à 75 mg de proanthocyanidines oligomères, 25 à 340 mg d'acide lipoïque, 5 à 75 mg de vitamine E, 0,25 à 5 mg de lycopène et 0,05 à 2,5 mg d'astaxanthine.

10. Composition antioxydante pour son utilisation selon la revendication 9, sous la forme d'une dose unitaire comprenant 5 à 25 mg de proanthocyanidines oligomères, 25 à 110 mg d'acide lipoïque, 5 à 25 mg de vitamine E, 0,25 à 1,5 mg de lycopène et 0,05 à 0,5 mg d'astaxanthine.

11. Composition antioxydante pour son utilisation selon la revendication 10, sous la forme d'une dose unitaire comprenant 6,7 mg de proanthocyanidines oligomères, 33 mg d'acide lipoïque, 6,7 mg de vitamine E, 0,4 mg de lycopène et 0,08 mg d'astaxanthine.

12. Composition antioxydante pour son utilisation selon la revendication 10, sous la forme d'une dose unitaire comprenant 20 mg de proanthocyanidines oligomères, 100 mg d'acide lipoïque, 20 mg de vitamine E, 1,2 mg de lycopène et 0,25 mg d'astaxanthine.

13. Composition antioxydante pour son utilisation selon la revendication 9, sous la forme d'une dose unitaire comprenant 20 à 55 mg de proanthocyanidines oligomères, 100 à 325 mg d'acide lipoïque, 20 à 55 mg de vitamine E, 1 à 4,5 mg de lycopène et 0,25 à 1,5 mg d'astaxanthine.
